# EUROPEAN PATENT SPECIFICATION

(11) **EP 1 524 007 B1**
(45) Date of publication and mention of the grant of the patent: **01.03.2017**
(21) Application number: 04256377.5
(22) Date of filing: 15.10.2004
(51) Int. Cl.: A61N 1/36

(54) **System for treating sleep apnea**
System zur Behandlung von Schlafapnoe
Système pour le traitement de l'apnea de sommeil

(30) Priority: 17.10.2003 US 512245 P
(43) Date of publication of application: 20.04.2005
(73) Proprietor: Alfred E. Mann Foundation for Scientific Research, Santa Clarita, CA 91380-9005 (US)
(72) Inventor: Davies, Ross, Rockport, MN 04856 (US); Zilberman, Yitzhak, Santa Clarita, CA 91321 (US); Cosendai, Gregoire, Cudrefin 1588 (CH)
(74) Representative: Carter, Stephen John

(56) References cited:
- EP-A- 0 404 427
- EP-A- 0 507 580
- EP-A- 0 702 977
- EP-A- 0 743 076
- US-A- 6 164 284

## Description

The present invention is generally directed to the application of implantable medical devices in treating physical dysfunctions, and more particularly to method and system for treatment of Obstructive Sleep Apnea (OSA).

### Background of the Invention

Obstructive sleep apnea (OSA) is a common disorder, estimated to affect 4% of middle-aged men and 2% of women. This disorder is characterized by recurrent pharyngeal collapse during sleep leading to repetitive arousals and nocturnal hypoxemia. OSA occurs in individuals with an anatomically small pharyngeal airway. Because of sleep-induced decreases in upper airway muscle tone, the airway collapses necessitating brief arousals from sleep to re-establish airway patency. These recurrent arousals result in sleep fragmentation, excessive daytime sleepiness and decreased quality of life. In addition, the arousals and the nocturnal hypoxemia result in catecholamine surges. Furthermore, there is accumulating evidence that adverse cardiovascular sequelae such as hypertension, arrhythmias, cerebrovascular events, myocardial infarction, and congestive heart failure may result from OSA.

Current methods of therapy are aimed at splinting the airway open during sleep (Continuous Positive Airway Pressure: CPAP) or enlarging the pharyngeal airway through surgery or the use of oral appliances. Nasal CPAP is the most effective and most widely used therapy for obstructive sleep apnea. However, acceptance of and compliance with CPAP creates limitations for its use. Compliance with CPAP is generally reported to be in the range of 50 to 85%. Patients who abandon CPAP therapy typically do so during the first 2 to 4 weeks of treatment because of issues related to mask discomfort, nasal dryness and congestion, and difficulty adapting to the pressure.

Studies demonstrate that compliance with CPAP is poor, although it can be improved with consistent follow-up (e.g. support groups) and when the patient's disease is severe. Untreated, patients with obstructive sleep apnea are at risk of negative sequelae. Thus, there exists a need for an alternative to CPAP for patients with obstructive sleep apnea.

US 6164284 describes a system for treating various conditions using nerve stimulation, the system including a plurality of microstimulators.

### Brief Description of the Drawings

FIG. 1 is an illustration of a system associated with a patient for treating OSA in accordance with the present invention.
FIG. 2 is an illustration of a block diagram of the system in accordance to the present invention.
FIG. 3 is a flow chart of the steps of a method of treating OSA in a patient.
FIG. 4 is an illustration of a microstimulator stimulating afferent and efferent fibers comprising the hypoglossal nerve originating in the brain stem and terminating in the tongue muscle in accordance to the present invention.
FIG. 5A is an illustration of alternating between left and right HGN efferent stimulation during inspiratory phases of a patient according to the present invention.
FIG. 5B is an illustration of both HGN efferent stimulation during inspiratory phases of a patient according to the present invention.
FIG. 5C is an illustration of both HGN efferent stimulation continuously during both inspiratory and expiratory phases of a patient according to the present invention.
FIG. 5D is an illustration of alternating between left and right but continuous HGN efferent stimulation during both inspiratory and expiratory phases of a patient according to the present invention.
FIG. 6A is an illustration of both HGN afferent stimulation continuously during both inspiratory and expiratory phases of a patient according to the present invention.
FIG. 6 B is an illustration of alternating between left and right but continuous HGN afferent stimulation during both inspiratory and expiratory phases of a patient according to the present invention.
FIG. 7 is a side view of a portion of a human anatomy showing the inside of the human mouth and schematically showing the positioning of a microstimulator close to one of the hypoglossal nerves.

### Detailed Description of the Preferred Embodiments

The following description is of the best mode presently contemplated for carrying out the invention which is defined in claim 1. Methods disclosed hereinafter do not form part of the scope of the invention.

This description is not to be taken in a limiting sense, but is made merely for the purpose of describing the general principles of the invention. The scope of the invention should be determined with reference to the claims.

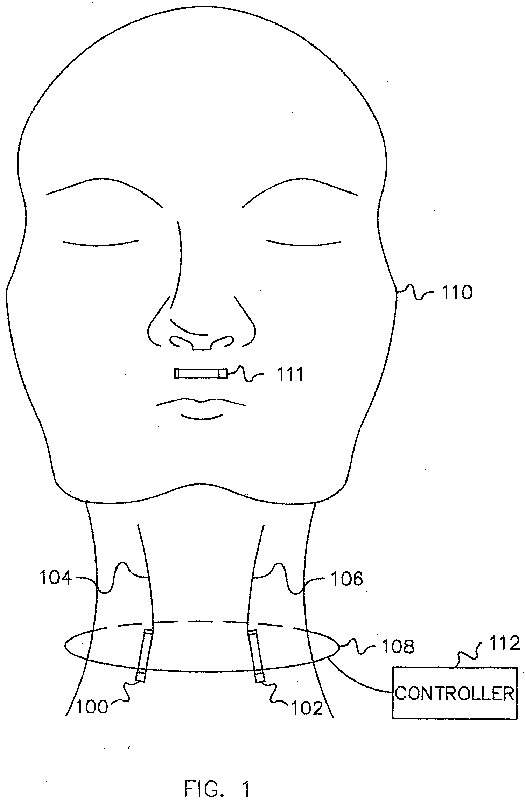

The present invention provides a system for treating OSA in a patient. As shown in FIG. 1, a patient exhibiting OSA is implanted with a pair of microstimulators, which are controllably activated to stimulate the left and right hypoglossal nerves (HGN) of the patient. It is known that traditionally a single electrode in the form of a nerve cuff electrode, percutaneous intramuscular motor-point electrode or implanted microstimulator has been used to stimulate only one of the HGNs or its motor-point in the genioglossus muscles (GGM) in a patient to cause the movement of the tongue in a desired direction in order to open the obstructed air way of a patient with OSA condition. The present invention contemplates providing one microstimulator implanted adjacent to each HGN in order to stimulate each HGN individually. There can be significant benefits in treating patients with OSA by providing one microstimulator adjacent to each left and right HGN.

As indicated above, the present invention is directed to a system for treating sleep apnea, and more particularly OSA using at least two small implantable microstimulators, also referred to as BION™ devices. Various features and details associated with the manufacture, operation and use of such implantable microstimulators, or BION devices, may be found in one or more of the following documents, all of which are incorporated herein by reference: U.S. Pat. No. 5,193,539 entitled "Implantable Microstimulator"; U.S. Pat. No. 5,193,540, entitled "Structure and Method of Manufacture of an Implantable Microstimulator"; U.S. Pat. No. 5,312,439 entitled "Implantable Device Having an Electrolytic Storage Electrode"; U.S. Pat. No. 6,164,284, entitled "System of implantable devices for monitoring and/or affecting body parameters; U.S. Pat. No. 6,185,452, entitled "Battery-powered patient implantable device"; U.S. Pat. No. 6,208,894, entitled "System of implantable devices for monitoring and/or affecting body parameters"; U.S. Pat. No. 6,315,721, entitled "System of implantable devices for monitoring and/or affecting body parameters"; 6,564,807, entitled "System of implantable devices for monitoring and/or affecting body parameters".

Referring to FIG. 1, it illustrates the system for treating OSA in accordance with the present invention. A pair of microstimulators 100 and 102 in the form of BION devices are subcutaneously provided proximate to the first and second HGNs 104 and 106 respectively. The BION devices are approximately less than 6 mm in lateral dimension and less than 60 mm in longitudinal dimension. In the embodiment of the present invention, it is contemplated that the BION devices are less than 2.5 mm in lateral dimension and less than 16 mm in longitudinal dimension. A power and data transmission coil 108 is placed in the vicinity of the neck or the chin of the patient 110. The coil 108 is connected to the controller 112. The controller 112 supplies the electrical power and data through the coil 108 to the microstimulators 100 and 102 via a wireless magnetic coupling. Furthermore, in the alternative, it is contemplated that the data communication can be effected via an electromagnetic coupling (not shown) of the microstimulators with the controller.

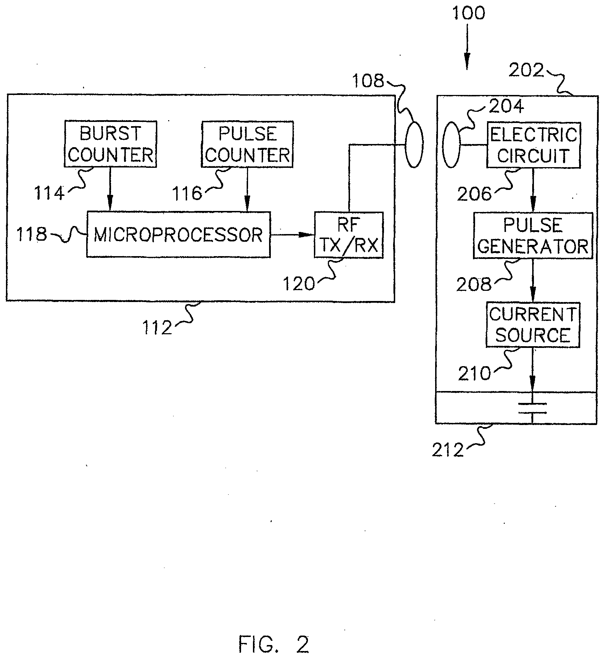

FIG. 2 illustrates a block diagram of the system in accordance to the present invention. Referring to FIG. 2, a typical microstimulator 100 or 102 comprises a biocompatible housing 202 having a coupling coil 204, an electrical circuitry 206 for power reception and data communication, a pulse generator 208 and current source 210 for providing electrical supply to the electrode 212 for stimulation. The controller 112 includes a burst counter 114, a pulse counter 116, a microprocessor 118, and an RF transmitter/receiver 120.

By increasing the GGM tone using cyclic/alternating stimulation to the HGNs, the posterior portion of the GGM will be more capable of contracting forward thereby relieving the airway obstruction. By alternating the stimulation of the left and right HGNs, each side of the GGM develops tone while there is a period of rest for the other side's GGM; this results in less chance of GGM fatigue. It is understood that a traditional approach of continuously stimulating and contracting only one side of the GGM results in muscle fatigue because the muscles require a relaxation period in order to allow blood circulation for oxygen and metabolic substances to reach the appropriate muscle fibers and for metabolic by-product to be removed. The alternating stimulation of the HGNs can be performed in equal or varying durations. For example, the right HGN can be stimulated for a first predetermined period of time and the left HGN can be stimulated for a second predetermined period of time. Each HGN stimulation period can be concurrent or sequential and based on distinct inspiratory phases of the patient. For example, it is contemplated to stimulate the first HGN based on the first inspiratory phase and to stimulate the second HGN based on the second inspiratory phase. Furthermore, the first predetermined period of time and the second predetermined period of time can be partially overlapping in time.

According to the present invention, the stimulation duration of each HGN based on the inspiratory phase is about 5 seconds, which is produced by the burst counter 114 and communicated to the microprocessor 118. Moreover, the frequency of the each stimulation pulse is the range of about 20 to 100 Hz (preferably about 30 Hz) and the pulse width is about 200 microseconds. The stimulation current is in the range of about 0.5 to 5 milliamps depending on the proximity of the microstimulator to the desired HGN. As shown in FIG. 2, the pulse counter 116 generates approximately a 30 Hz signal for transmission to BION microstimulator. The microprocessor 118 communicates the desired stimulation pulsing data and power through the RF transmitter/receiver 120 via the coil 108 to the coupling coil 204 of the BION device.

The stimulation timing of both HGNs can be either by a closed-loop feedback from sensors or an open-loop method. In an embodiment of an open-loop method, a patient's breathing pattern is monitored while asleep. The monitoring is accomplished either with another person present or through any known medical monitoring equipment that may be connected to the patient. The phase of the breathing cycle/ respiratory function of the patient may be monitored by a monitoring unit in the form of sensor such as a nasal flow probe 111, thermocouple, a pulse oxygen sensor, or with force sensitive bands on the thorax, or with any other monitoring means known to those skilled in the art. After the monitoring of the patient's breathing pattern, a determination is made as to the length and frequency of the patient's inspiratory effort/pattern. Having the aforementioned information available, then alternating stimulation to each HGN to cause alternating contraction of each side of the GGM according to the inspiratory pattern is performed.

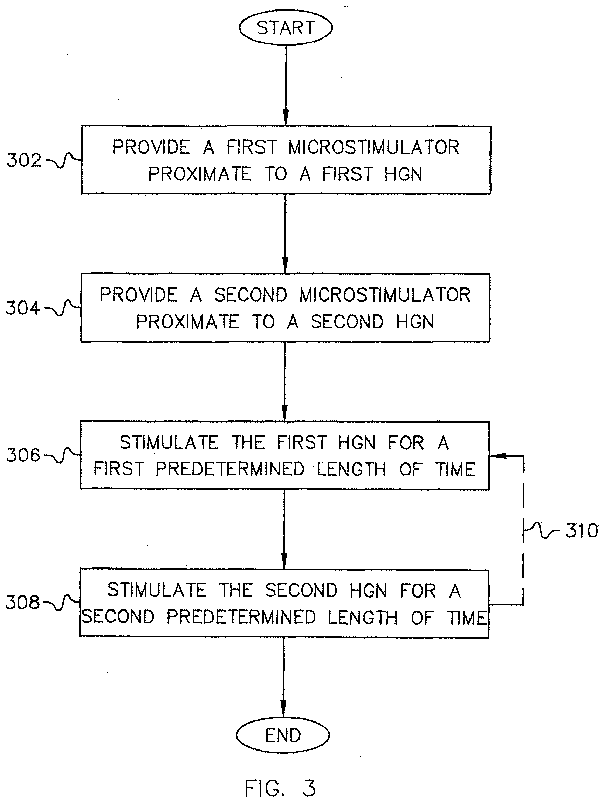

FIG. 3 is a flow chart of the steps of a method of treating OSA in a patient. Initially, the first microstimulator is provided proximate to the first HGN (302); and secondly, the second microstimulator is provided proximate to the second HGN (304). Thereafter, the first HGN is stimulated for a first predetermined length of time (306) and the second HGN is stimulated for a second predetermined length of time (308). In order to maintain the air passage open during the inspiration phase of the respiratory pattern of the patient, stimulation of the first and the second HGNs are repeated as shown by a dotted line (310).

In an embodiment of a closed-loop method, a sensor or a group of sensors are utilized to monitor the patient's respiratory function similar to those sensors utilized in the open-loop method. The sensors can be any conventional sensors (e.g. those positioned on the nose or surface of the skin of the thorax/abdomen of a patient) capable of monitoring parameters of a patient indicative of the respiratory function. Hence, utilizing the sensors the respiratory function of the patient is monitored such that based on the determination of the inspiratory pattern one or both of the HGNs of the patient are stimulated. A feedback control signal is provided from the sensors monitoring the respiratory cycle/function of the patient to the controller, which in turn controls the operation of the stimulation function of the microstimulators.

In an aspect of the present invention, it is contemplated that instead of alternating/cycling, both microstimulators would stimulate the left and the right HGNs concurrently. When both sides of the GGMs contract at the same time, each side would then need less contraction than when one side is used. This will be advantageous in providing a lower amount of stimulation current to each HGN than the total amount of stimulation current applied to a single HGN in a traditional OSA treatment, and possibly provide a more symmetrical opening of the pharynx. This mode of simultaneous HGN stimulation could be during inspiratory phases only or during both inspiratory and expiratory phases of a sleeping patient.

In normal individuals (i.e., non-apneic), the level of activity of the GGM is the same during sleep as it is during wakefulness. In individuals with OSA, the level of activity of the GGM is increased approximately three times normal during wakefulness to compensate for their structurally small pharyngeal airway. When asleep, individuals with OSA experience a large reduction in genioglossus activity from the elevated levels seen when they are awake. In the present invention, it is contemplated that an afferent stimulation of the HGN is provided, which is set at a sub-motor threshold level. This is intended to cause an increase in the excitability of the hypoglossal nucleus (brain stem) resulting in an increase in firing of motor axons down the HGN producing an increase in tone of GGM muscles, thereby leading to a sustained increase in the pharyngeal airway and thus alleviating the main problem of OSA.

In the present invention, it is contemplated that the afferent fibers of the left and right HGN(s) are stimulated concurrently or in an alternate fashion. Furthermore, it is contemplated in the present invention that a motor-threshold level stimulation of the efferent fibers of the HGN(s) is provided as an excitatory input to the GGM(s). This is intended to produce an opening of the airway, thereby relieving an obstruction of the OSA.

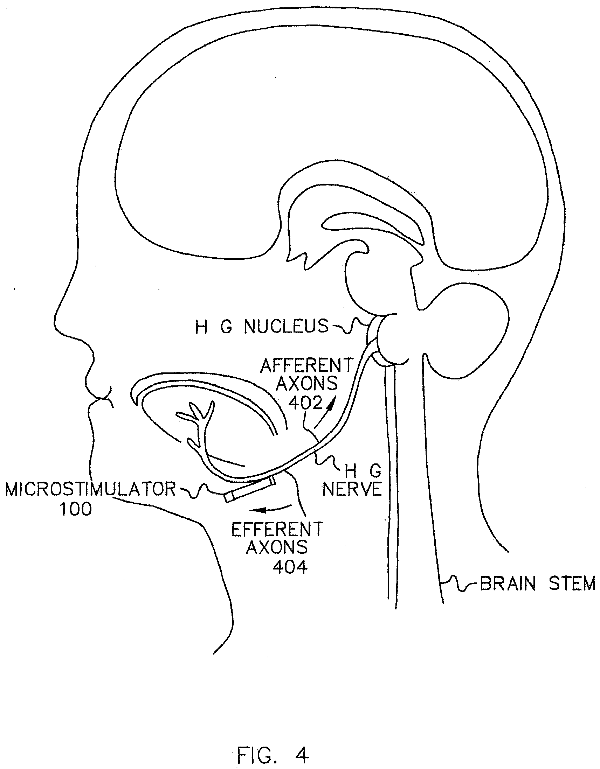

FIG. 4 is an illustration of a microstimulator stimulating afferent and efferent fibers comprising the hypoglossal nerve originating in the brain stem hypoglossal nucleus and terminating in one side of the tongue's GGM. As shown in FIG. 4, the microstimulator 100 can provide either an afferent stimulation (sub-motor threshold) or an efferent stimulation (at or above-motor threshold) to the respective afferent 402 and efferent 404 fibers (axons) of the HGN.

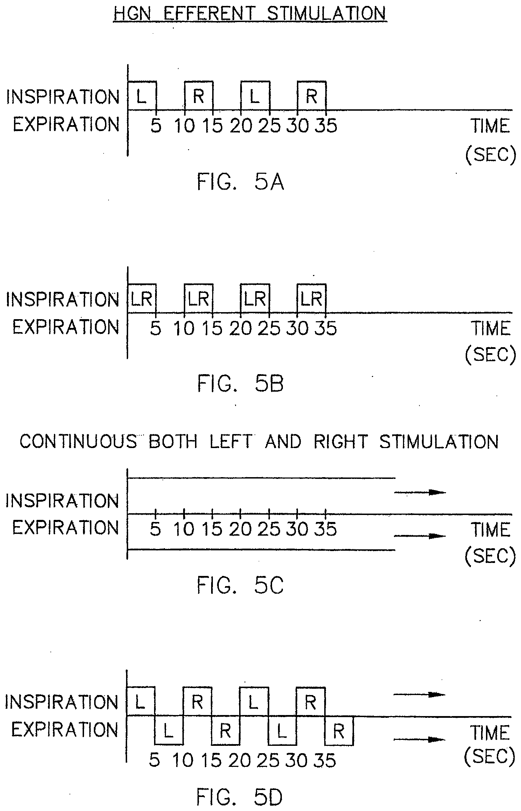

The various operations and stimulation methods according to the present invention are described below with reference to the relevant figures-FIG. 5A through FIG. 6B:

Referring to FIG. 5A, it is contemplated that the HGNs are stimulated at efferent stimulation level by alternating between left and right HGNs during the inspiratory phases of the patient.

Referring to FIG. 5B, it is contemplated that the HGNs are stimulated at efferent stimulation level by stimulating both left and right HGNs concurrently during the inspiratory phases of the patient.

Referring to FIG. 5C, it is contemplated that the HGNs are stimulated continuously at efferent stimulation level by stimulating both left and right HGNs concurrently during the inspiratory and expiratory phases of the patient.

Referring to FIG. 5D, it is contemplated that the HGNs are stimulated continuously at efferent stimulation level by alternately stimulating the left and then the right HGNs during the inspiratory and expiratory phases of the patient and vice versa.

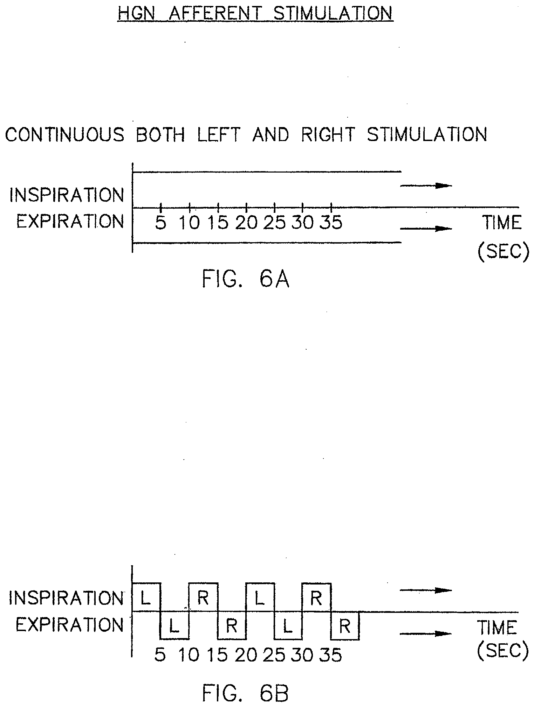

Referring to FIG. 6A, it is contemplated that the HGNs are stimulated continuously at afferent stimulation level by stimulating both left and right HGNs concurrently during the inspiratory and expiratory phases of the patient.

Referring to FIG. 6B, it is contemplated that the HGNs are stimulated continuously at afferent stimulation level by alternately stimulating the left and then the right HGNs during the inspiratory and expiratory phases of the patient and vice versa.

It must be noted that the aforementioned operations and stimulation methods may be effected utilizing a closed-loop or an open-loop method as described above.

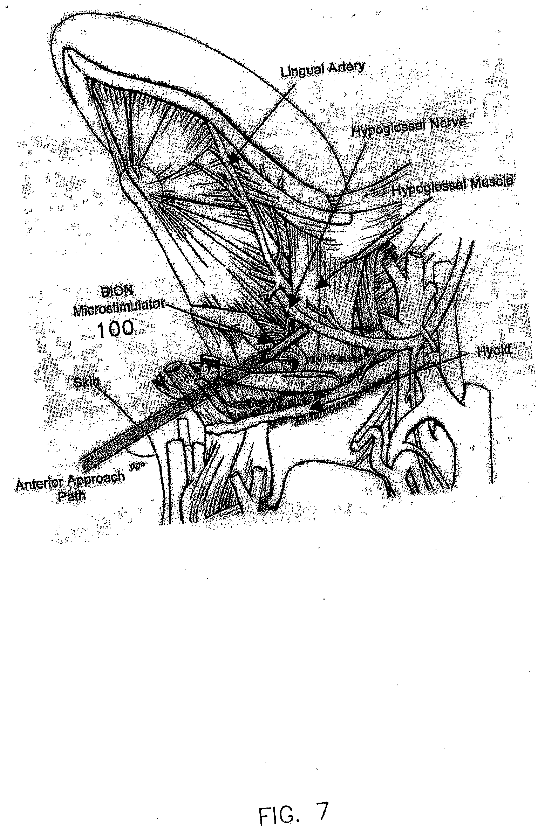

For a more clear visualization of the positioning of the microstimulators with respect to the HGN, FIG. 7 is presented. FIG. 7 is a side view of a portion of a human anatomy showing the inside of the human mouth and schematically showing the positioning of a microstimulator close to one of the HGNs. The microstimulator can be positioned in the intended location using an anterior approach path.

The descriptions of the invention, the specific details, and the drawings mentioned above, are not meant to limit the scope of the present invention. The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes, which come within the meaning and range of equivalency of the claims, are to be embraced within their scope.

## Claims

1. A system for treating sleep apnea in a patient comprising:
a first microstimulator adapted to stimulate a first hypoglossal nerve (HGN);
a second microstimulator adapted to stimulate a second HGN; and
a controller for controlling the stimulation function of the first and the second microstimulators;
wherein the first microstimulator is adapted to stimulate the first HGN for a first predetermined length of time and the second microstimulator is adapted to stimulate the second HGN for a second predetermined length of time, and the first predetermined length of time and the second predetermined length of time occur sequentially or alternatingly.

2. The system of claim 1, wherein each of the first and the second microstimulators are less than 60 mm in longitudinal and less than 6 mm in lateral dimension.

3. The system of claim 1, or 2, wherein the first microstimulator and the second microstimulator are controlled by the controller through a wireless communication medium.

4. The system of claim 3, wherein the wireless communication medium is electromagnetic communication.

5. The system of claim 3, wherein the wireless communication medium is magnetic communication.

6. The system of any one of claims 1 to 5, further comprising a monitoring unit for monitoring the respiratory function of the patient.

7. The system of claim 6, wherein the monitoring unit includes at least one sensor.

8. The system of claim 6 or 7 wherein the monitoring unit provides a feedback control signal to the controller for controlling the stimulation function of the first and the second microstimulators.

9. The system of any one of claims 1 to 8, wherein the first microstimulator and the second microstimulator are adapted to provide stimulation at a sub-motor threshold afferent stimulation level.

10. The system of any one of claims 1 to 9, wherein the first microstimulator and the second microstimulator are adapted to provide stimulation at a motor-threshold level.

11. The system of claim 1 or any claim dependent on claim 1, wherein the first predetermined length of time and the second predetermined length of time can be partially overlapping in time.

12. The system of claim 1 or any claim dependent on claim 1, wherein the first predetermined length of time is based on the first inspiratory phase of the patient.

13. The system of claim 12, wherein the second predetermined length of time is based on the second inspiratory phase of the patient.

14. The system of claim 1, wherein the sequential stimulation is performed continuously.

## Patentansprüche

1. System zur Behandlung von Schlafapnoe in einem Patienten, umfassend:
einen ersten Mikrostimulator, der zur Stimulierung eines ersten Unterzungennervs (HGN) ausgelegt ist;
einen zweiten Mikrostimulator, der zur Stimulierung eines zweiten HGN ausgelegt ist; und
eine Steuerungseinheit für das Steuern der Stimulierungsfunktion des ersten und des zweiten Mikrostimulators;
worin der erste Mikrostimulator zur Stimulierung des ersten HGN für eine erste vorbestimmte Zeitdauer ausgelegt ist und der zweite Mikrostimulator zur Stimulierung des zweiten HGN für eine zweite vorbestimmte Zeitdauer ausgelegt ist, und die erste vorbestimmte Zeitdauer und die zweite vorbestimmte Zeitdauer aufeinanderfolgend oder abwechselnd erfolgen.

2. System nach Anspruch 1, worin jeder des ersten und des zweiten Mikrostimulators ein Längsausmaß von weniger als 60 mm, und ein Querausmaß von weniger als 6 mm aufweist.

3. System nach Anspruch 1 oder 2, worin der erste Mikrostimulator und der zweite Mikrostimulator durch die Steuerungseinheit durch ein drahtloses Kommunikationsmedium gesteuert werden.

4. System nach Anspruch 3, worin das drahtlose Kommunikationsmedium elektromagnetische Kommunikation ist.

5. System nach Anspruch 3, worin das drahtlose Kommunikationsmedium magnetische Kommunikation ist.

6. System nach einem der Ansprüche 1 bis 5, ferner umfassend eine Überwachungseinheit zur Überwachung der Atemfunktion des Patienten.

7. System nach Anspruch 6, worin die Überwachungseinheit mindestens einen Sensor umfasst.

8. System nach Anspruch 6 oder 7, worin die Überwachungseinheit der Steuerungseinheit ein Rückkopplungssteuersignal zum Steuern der Stimulationsfunktion des ersten und des zweiten Mikrostimulators bereitstellt.

9. System nach einem der Ansprüche 1 bis 8, worin der erste Mikrostimulator und der zweite Mikrostimulator ausgelegt sind, Stimulation auf einem sub-motorischen Schwellenwert-afferenten Stimulationsniveau bereitzustellen.

10. System nach einem der Ansprüche 1 bis 9, worin der erste Mikrostimulator und der zweite Mikrostimulator zur Bereitstellung von Stimulierung auf einem motorischen-Schwellenwertniveau ausgelegt sind.

11. System nach Anspruch 1 oder einem beliebigen, von Anspruch 1 abhängigen Anspruch, worin die erste vorbestimmte Zeitdauer und die zweite vorbestimmte Zeitdauer sich zeitlich teilweise überlappen können.

12. System nach Anspruch 1, oder einem beliebigen, von Anspruch 1 abhängigen Anspruch, worin die erste vorbestimmte Zeitdauer auf der ersten Einatmungsphase des Patienten basiert.

13. System nach Anspruch 12, worin die zweite vorbestimmte Zeitdauer auf der zweiten Einatmungsphase des Patienten basiert.

14. System nach Anspruch 1, worin die aufeinanderfolgende Stimulierung kontinuierlich ausgeführt wird.

## Revendications

1. Système pour traiter l'apnée du sommeil chez un patient, comprenant :
un premier micro-stimulateur adapté pour stimuler un premier nerf hypoglosse (HGN) ;
un second micro-stimulateur adapté pour stimuler un second HGN ;
et une commande pour commander la fonction de stimulation des premier et second micro-stimulateurs ;
dans lequel le premier micro-stimulateur est adapté pour stimuler le premier HGN pendant une première durée prédéterminée et le second micro-stimulateur est adapté pour stimuler le second HGN pendant une seconde durée prédéterminée, et la première durée prédéterminée et la seconde durée prédéterminée surviennent séquentiellement ou alternativement.

2. Système selon la revendication 1, dans lequel chacun des premier et second micro-stimulateurs a une dimension longitudinale inférieure à 60 mm et une dimension latérale inférieure à 6 mm.

3. Système selon la revendication 1 ou 2, dans lequel le premier micro-stimulateur et le second micro-stimulateur sont commandés par la commande par l'intermédiaire d'un support de communication sans fil.

4. Système selon la revendication 3, dans lequel le support de communication sans fil est une communication électromagnétique.

5. Système selon la revendication 3, dans lequel le support de communication sans fil est une communication magnétique.

6. Système selon l'une quelconque des revendications 1 à 5, comprenant en outre une unité de surveillance pour surveiller la fonction respiratoire du patient.

7. Système selon la revendication 6, dans lequel l'unité de surveillance comprend au moins un capteur.

8. Système selon la revendication 6 ou 7, dans lequel l'unité de surveillance fournit un signal de commande de rétroaction à la commande pour commander la fonction de stimulation des premier et second micro-stimulateurs.

9. Système selon l'une quelconque des revendications 1 à 8, dans lequel le premier micro-stimulateur et le second micro-stimulateur sont adaptés pour fournir une stimulation à un niveau de stimulation afférent au seuil de sous-moteur.

10. Système selon l'une quelconque des revendications 1 à 9, dans lequel le premier micro-stimulateur et le second micro-stimulateur sont adaptés pour fournir une stimulation à un niveau de seuil de moteur.

11. Système selon la revendication 1 ou toute revendication dépendante de la revendication 1, dans lequel la première durée prédéterminée et la seconde durée prédéterminée peuvent se chevaucher partiellement dans le temps.

12. Système selon la revendication 1 ou toute revendication dépendante de la revendication 1, dans lequel la première durée prédéterminée est basée sur la première phase inspiratoire du patient.

13. Système selon la revendication 12, dans lequel la seconde durée prédéterminée est basée sur la seconde phase inspiratoire du patient.

14. Système selon la revendication 1, **caractérisé en ce que** la stimulation séquentielle est réalisée en continu.
